(19)

Europäisches Patentamt
European Patent Office
Office européen des brevets

(11)     EP 2 374 507 A1

(12)     EUROPEAN PATENT APPLICATION

(43) Date of publication:
12.10.2011 Bulletin 2011/41

(51) Int Cl.:
*A61P 3/04* (2006.01)     *A23L 1/29* (2006.01)
*A23L 1/304* (2006.01)     *A23L 1/305* (2006.01)
*A61K 35/20* (2006.01)     *A61K 38/00* (2006.01)
*A61K 38/55* (2006.01)

(21) Application number: 10011956.9

(22) Date of filing: 25.02.2003

(84) Designated Contracting States:
AT BE BG CH CY CZ DE DK EE ES FI FR GB GR HU IE IT LI LU MC NL PT SE SI SK TR

(30) Priority: 01.03.2002 US 360709 P
21.02.2003 US 371534 P

(62) Document number(s) of the earlier application(s) in accordance with Art. 76 EPC:
03709343.2 / 1 483 023

(71) Applicant: Glanbia Nutritionals (Ireland) Limited
Kilkenny (IE)

(72) Inventors:
• Ward, Loren, S.
Twin Falls, ID 83301 (US)

• Bastian, Eric, D.
Twin Falls, ID 83301 (US)
• Paulsen, Starla, J.
Twin Falls, ID 83301 (US)

(74) Representative: Golding, Louise Ann
Dehns
St Bride's House
10 Salisbury Square
London
EC4Y 8JD (GB)

Remarks:
•This application was filed on 30-09-2010 as a divisional application to the application mentioned under INID code 62.
•Claims filed after the date of filing of the application (Rule 68(4) EPC).

(54) Compositions and methods for treatment of body weight conditions with milk minerals, casein fractions and enzyme inhibitor

(57)     A nutritional supplement composition having therapeutically effective amounts of milk minerals including calcium, a protein source including κ-casein fragment 106-169, and enzyme-inhibiting peptides is provided for the treatment of body weight conditions. The nutritional supplement composition is administered in amounts effective for limiting weight gain and/or enhancing weight loss, as well as promoting overall good health, in the treatment of body weight conditions, including overweight and obesity.

EP 2 374 507 A1

**Description**

CROSS REFERENCE TO RELATED APPLICATIONS

This application is based on, and claims the benefit of co-pending United States Provisional Application Serial No. 60/360,709, filed on March 1, 2002, and entitled 'Compositions and Methods for Treatment of Obesity', and co-pending United States Application filed on February 21, 2003, entitled 'Compositions and Methods for Treatment of Body Weight Conditions', the disclosures of which are incorporated herein by reference.

BACKGROUND OF THE INVENTION

The present invention is directed to compositions and methods for treatment of body weight conditions by administering a therapeutically effective nutritional supplement composition. More particularly, the nutritional composition, which includes a milk mineral blend and protein components, is effective for enhancing weight loss and/or limiting weight gain.

Obesity is a significant worldwide health concern that affects both young and old. In the United States, it is estimated that more than 50% of men and women are overweight. The degree to which a person may be overweight can be evaluated based on the person's "body mass index" or "BMr', which is calculated as follows:

$$\mathrm{BMI} = \text{Weight in kilograms (kg)/[Height in meters (m)]}^{2}.$$

In 2000, almost 20% of the population fell into the obese category as defined by a BMI of greater than or equal to 30. Problems associated with obesity include cardiovascular disease, diabetes mellitus, certain types of cancer, osteoarthritis and sleeping disorders. Obesity and related disorders account for almost 10% of US health care expenditures.

Over 90% of body energy is stored in adipose (fat) tissue. Adipose tissue has a number of important functions, the most obvious of which is to "buffer" the daily influx of dietary fat entering the blood circulation and release fatty acids as a source of metabolic fuel when needed. Other functions include the production and release of adipsin (essential for blood clotting), angiotensinogen (involved in blood pressure control), and leptin (a hormone involved in energy control). An accumulation of adipose tissue leads to overweight and obesity.

Diet is known to have effects on weight control. Excesses in diet, such as high caloric intake and consumption of high fat foods, can result in undesired weight gain and poor health. Similarly, a diet lacking one or more nutrients also can have a negative impact on weight control and health. For example, literature suggests that that a diet deficient in calcium can contribute to the occurrence obesity. Shi et al., "Effects of dietary calcium on adipocyte lipid metabolism and body weight regulation in energy-restricted aP2-agouti transgenic mice" FASEB J. 15(2), 291-93 (2001); Zemel et al., "Regulation of adiposity by dietary calcium," FASB J. 14(9) 1132-38 (2000).

Mechanisms in the human body also are known to impact weight gain. For example, when food is consumed, the body releases a peptide, cholecystokinin (CCK), which acts to signal satiety as a result of promoting secretion of enzymes and other bodily fluids and other physical reaction within the gastric system. It has been shown that CCK release results in appetite reduction so that the person will stop eating. Proteins such as κ-casein fragment 106-169, also referred to as glycomacropeptide (GMP), are known to stimulate the release of CCK.

Various weight control compositions and methods for use by overweight and obese adults are known. Typically, however, the methods focus on a particular weight control mechanism in order to control weight gain or promote weight loss, with little or no regard for providing a balanced diet. For example, U.S. Patent No. 6,384,087 to Zemel discloses methods and materials for treating or avoiding obesity in humans and other animals. The patent discloses that the obesity-control benefits can be achieved by providing a diet high in calcium. Additionally, the patent teaches that individuals are maintained on a restricted caloric diet. The weight control mechanism of this patent is directed to providing increased levels of one specific nutrient, calcium, while optionally limiting caloric intake, without promoting a balanced diet. As another example, U.S. Patent No. 6,207,638 to Portman discloses a nutritional intervention composition for enhancing and extending satiety by stimulating the release of CCK. The composition includes a protein, a glycomacropeptide, long chain fatty acids, calcium (in the form of calcium carbonate or calcium lactate) and a combination of soluble and insoluble fibers. The patent teaches that the composition can be taken orally to permit a person to be satiated with a lower calorie intake. The weight management mechanism of this composition is directed to limiting caloric intake, without addressing overall nutritional requirements.

There remains a need for methods of improving human diets in order to maintain an ideal weight, reduce weight gain and/or enhance weight loss while promoting a nutritionally balanced diet. There also remains a need for dietary compositions to provide essential nutrients associated with a healthy diet to reduce incidence of overweight and obesity and maintain overall health.

SUMMARY OF THE INVENTION

The present invention is directed to compositions for the management of body weight and treatment of body weight conditions, such as overweight or obesity. According to one aspect of the invention, a weight control composition is provided that includes one or more of a milk mineral blend in an amount effective for decreasing adiposity, a protein compound in an amount effective for enhancing satiety after consumption of food, and enzyme-inhibiting peptides in an amount effective for controlling fat metabolism.. The milk mineral blend includes calcium, and the protein compound includes κ-casein fragment 106-169. According to another aspect of the invention, a nutritional supplement for maintaining and/or reducing weight is provided. The supplement can include a milk mineral composition having calcium in an amount effective for decreasing adiposity. The supplement also can include a protein composition having κ-casein fragment 106-169 in an amount effective for enhancing satiety to limit or curtail consumption of food. The supplement also can include enzyme-inhibiting peptides, such as angiotensin converting enzyme- inhibiting peptides, in an amount effective for enhancing weight loss in treating an overweight condition or obesity.

According to yet another aspect of the invention, methods of maintaining a desired weight and treating an overweight condition or obesity include administering to an individual in need of such treatment a nutritional composition limiting weight gain and/or enhancing weight loss, as well as promoting overall good health. The composition can include therapeutically effective amounts of a milk mineral component, a protein component and/or an enzyme-inhibiting peptide component. The composition can be taken directly by an individual or administered via a food product fortified with the composition. The amount of nutritional supplement composition when administered via a food product can be suitably selected, for example, such that a daily serving or a predetermined number of servings of the food product delivers an amount of the composition effective for maintaining a desired weight or treating an overweight condition or obesity. The composition can be administered just prior to or after consumption of food, as part of a meal or as a snack between meals.

The nutritional supplement composition, generally in the form of a powder of appropriate particle size, can be incorporated into a wide variety of types of food products. By way of example, the nutritional composition can be added to acidic juice beverages (e.g., orange juice, apple juice, grape juice, grapefruit juice, cranberry juice, or blended juices), acidic beverages (e.g., sport beverages, Gatorade®), neutral pH beverages (e.g., milk UHT dairy, RTD nutritional, soy milk, or shakes and other blended beverages such as milkshakes, smoothies, frappes), nutritional supplement foodstuffs (e.g., high-energy protein bars), confectionery products (e.g., high calcium chews, chewing gum, chocolate, or cookies), dairy products (e.g., yogurt, ice cream, milk, cheese, processed cheese, or butter), and farinaceous products (e.g., bread, muffins, biscuits, cereal or rolls). Alternatively, the nutritional supplement composition can be administered directly, such as in the form of tablets or capsules and optionally combined with other minerals and/or vitamins.

DETAILED DESCRIPTION OF THE INVENTION

The present invention is directed to compositions and methods for maintaining a predetermined body weight range and treating an overweight condition or obesity by enhancing weight loss and/or limiting weight gain and promoting good health. Overweight and obesity has been associated to some degree with inadequate intake of dairy products, and more particularly the minerals present in dairy products. It has been discovered that an overweight condition and obesity can be effectively treated by administering nutritional supplement compositions, either directly or via food products fortified with the compositions, in accordance with the practice of the present invention. The nutritional supplement compositions contain therapeutically effective amounts of milk mineral, protein and enzyme-inhibiting peptides and are administered prior to or during a meal. The nutritional supplement compositions also can be administered to an individual seeking to maintain a desired body weight.

Treatment can be enhanced by use of additional ingredients in the composition to address other mechanisms for weight control. The compositions can include κ-casein fragment 106-169 (or a source of such peptides) to limit caloric intake by providing a sense of satiety, which will lead to termination of eating. Additionally, enzyme-inhibiting peptides may be included to assist with regulation of adiposity by controlling fat metabolism.

The terms "treat," "treating," "treatment," and similar terms as used herein refer to the administration of the nutritional supplement compositions to individuals, particularly humans, who are overweight or obese, for alleviating, suppressing, inhibiting, or otherwise reducing the extent to which the individual is overweight or obese or any symptom associated therewith. The terms "treat," "treating," "treatment," and similar terms also are used herein to refer to the prophylactic administration of the nutritional supplement compositions to individuals who may be at risk of, or otherwise wish to avoid, becoming overweight or obese.

One component of the nutritional supplement composition is milk minerals. The term "milk mineral," as used herein, refers to a mineral complex obtained from whey or milk. The mineral complex contains a balanced form of calcium, copper, magnesium, phosphorus, potassium, selenium and zinc. Milk mineral has a relatively neutral taste, in contrast to the chalky taste of calcium carbonate. Whey fractions that are high in calcium have been demonstrated to exhibit higher calcium bioavailability than are exhibited by calcium carbonate and calcium lactate. Ranhotra et al., "Bioavailability of Calcium in a High Calcium Whey Fraction," Nutrition Research, Vol. 17 Nos. 11-12, pp. 1663-1670 (1997). For optimal absorption, calcium and phosphorous preferably are present in a calcium-to-phosphorous ratio of about 1:1 to 2:1, e.g., a ratio similar to that found in both milk and in bone. The milk mineral also typically contains quantities of lactose and bioactive proteins. Milk mineral is also commonly referred to as "milk calcium."

Milk mineral provides various benefits as compared to supplements having other forms of calcium. Calcium supplements and calcium-fortified foods contain calcium in such forms as calcium carbonate, calcium lactate, calcium citrate, calcium chloride, and calcium hydroxide. These forms of calcium, however, can yield undesirable flavors and/or can strip desirable aroma and flavor compounds from food products. Use of milk minerals can avoid these problems. More significantly, the milk mineral complex delivers not only calcium but a balanced and pure form of the other milk minerals, including calcium, copper, magnesium, phosphorus, potassium, selenium and zinc, that are present only in milk and dairy products and that are important to a healthy diet. As a result, the milk mineral complex provides a balanced form of minerals, including calcium that is a preferred form of calcium and other minerals from a nutritional standpoint.

Suitable methods of obtaining milk mineral by extraction from whey or milk are known to persons skilled in the art. One suitable extraction method is described in U.S. Patent 5,639,501, the disclosure of which hereby is incorporated by reference in its entirety. Additionally, commercially available milk mineral products include TRUCAL® products, which are commercially available from Glanbia Nutritionals, Inc. of Monroe, Wisconsin. A typical composition of milk mineral is illustrated in Table 1 below.

*Table 1- Typical Composition for Milk Mineral Powder*

| Component | Relative Amount (% by weight) |
|---|---|
| Total Minerals | 50-90% |
| Inorganic Mineral (Ash) | 45-85% |
| Organic Mineral (Citrate) | 1-10% |
| Calcium | 15-35% |
| Magnesium | 0-10% |
| Phosphorous | 7-15% |
| Potassium | 0-5% |
| Zinc | 0-1% |
| Lactose | 0-15% |
| Protein | 1-15% |
| Free Moisture | 2-5% |
| Fat | 0-5% |

One important attribute of milk mineral is the calcium-to-magnesium ratio. Predetermined calcium-to-magnesium ratios are desired to limit or avoid leaching of other important minerals, which in turn may lead to bone brittleness and can even increase the risk of osteoporosis. Without wishing to be bound by any theories, high dietary Ca:Mg ratios interfere with magnesium absorption because calcium and magnesium share common intestinal absorption pathways. When calcium levels are high with respect to magnesium levels, calcium competes with magnesium for the absorption pathways, resulting in hypomagnesaemia (low magnesium in the blood).

The natural milk minerals, especially calcium, copper, magnesium, phosphorus, potassium, selenium and zinc, are of great importance in nutrition. Calcium, for example, is essential to many body functions, such as muscle function regulation, blood clotting, hormone regulation, nerve function, and enzyme activation. Calcium in milk mineral has a high bioavailability, which is enhanced by vitamin D, lactose, gastrointestinal acidity, and certain fibers. Also, the balanced form of calcium, copper, magnesium, phosphorus, potassium, selenium and zinc, and vitamin D in milk mineral helps to minimize calcium depletion through urinary loss.

The balance of minerals and bioactive proteins in the milk mineral renders food products fortified with the compositions of the present invention effective for healthy weight maintenance and in the treatment of the conditions of overweight and obesity. While not wanting to be bound by any theory, the following provides a discussion of the various mechanisms by which body weight conditions can be treated using the compositions of the present invention.

The milk minerals of the present compositions provide high calcium bioavailability effective for managing body weight and treating conditions including overweight and obesity. A common metabolic defect in cellular calcium ion handling is thought to contribute to the occurrence of weight gain and obesity. Low calcium intake increases intracellular calcium concentration in the adipocyte (fat cell) thereby switching its metabolism from lipolysis (fat breakdown) to lipogenesis (fat synthesis) and fat accumulation. By increasing the amount of calcium intake, intracellular calcium concentration is reduced, which leads to increased lipolysis and decreased lipogenesis. Thus, it is desired to provide a daily intake of an amount of calcium effective for weight maintenance and/or loss through reduction of fat tissue mass.

In another aspect of the present invention, the nutritional supplement composition includes a protein source such as whey proteins or other suitable food protein. Whey proteins occur in milk as soluble, globular proteins. Generally, they are an important source of protein needed for overall good health and nutrition. The primary proteins and peptide constituents derived from whey proteins include α-lactalbumin and β-lactoglobulin, κ-casein fragment 106-109, lactoferrin, bovine serum albumin, lactoperoxidase, and immunoglobulins.

An important peptide constituent is κ-casein fragment 106-109. These peptides function as an appetite suppressant by stimulating the release of the gastrointestinal hormone CCK. CCK is effective for short-term control of eating behavior because it generates responses in the body that are associated with satiety, thereby resulting in termination of the meal. Thus, by administering an effective amount of κ-casein fragment 106-169 prior to, during, or even shortly after a meal, the amount of food eaten during a meal can be limited while providing a sense of satiety. Additionally, administering κ-casein fragment 106-169 between meals when a person may feel hungry may also provide a sense of satiety, thereby avoiding undesired snacking between meals.

Sources of κ-casein fragment 106-169 include PROVON® 190 and PROVON® 290, which are commercially available from Glanbia Nutritionals, Inc. of Monroe, Wisconsin. Suitable methods for producing κ-casein fragment 106-169 are known to those of skill in the art and are, for example, described in U.S. Patent Nos. 5,278,288 and 5,280,107, incorporated herein by reference in their entirety, which describe processes for producing κ-casein fragment 106-169 from milk raw materials such as cheese whey and whey protein concentrates.

In another aspect of the present invention, the composition includes enzyme-inhibiting peptides. Sources of such peptides include casein, whey proteins, soy proteins, or any other suitable commercially available food protein which can be processed according to any methods known to those of skill in the art to provide the peptides. One example of such peptides are those that inhibit angiotensin converting enzyme (ACE). Angiotensin II is a hormone that is synthesized and secreted by adipose cells. Literature has shown that angiotensin II may be involved in control of adiposity through regulation of lipid synthesis and storage of adipocytes. Some dairy peptides are associated with the inhibition of angiotensin converting enzyme (ACE). Thus, by administering a therapeutically effective amount of ACE-inhibiting peptides, weight loss can be enhanced. Another type of enzyme-inhibiting peptides are those that will inhibit the enzymatic breakdown of CCK. One or more enzyme-inhibiting peptides can be included in the composition.

To obtain the compositions of the present invention, the components selected for the compositions can be processed as desired prior to preparation of the nutritional supplement compositions. Milk mineral extract typically is purified, spray dried, and ground into a powder having an appropriate particle size to permit mixing with a liquid or solid food product if desired. The milk mineral extract has calcium and other minerals as shown in Table 1. Similarly, the protein component typically is purified, dried, and ground into a powder. The protein component has κ-casein fragment 106-169 and, if desired, amino acids and other nutrients essential for overall good health and nutrition. ACE-inhibiting peptides also can be provided in the nutritional supplement composition. The desired components, which are selected from milk mineral extract, protein component and ACE-inhibiting peptides and combinations thereof, are blended to provide the nutritional supplement compositions of the present invention. The composition optionally can include other ingredients, such as minerals, vitamins, flavorings and colorants, in accordance with techniques well known to persons skilled in the art.

Suitable particle sizes for the composition will depend on such factors as the physical properties (e.g., liquid or solid, specific gravity, pH, viscosity, etc.) of the food product into which the powder is mixed. The mean particle size most often ranges from about 0.1 microns to about 300 microns, more usually from about 1 micron to about 100 microns. For neutral pH beverages, such as milk, a more finely ground powder preferably is employed so that a suspension of the powder can be easily formed. Because the solubility of the powder increases as pH decreases, less finely ground powders typically can be used, for example, in acidic juice beverages and in acidic beverages, in which the powder solubilizes.

The nutritional supplement composition in powder form can be used as an additive for a wide variety of types of food products, including acidic juice beverages (e.g., orange juice, apple juice, grape juice, grapefruit juice, cranberry juice, or blended juices), acidic beverages (e.g., sport beverages, Gatorade®), neutral pH beverages (e.g., milk UHT dairy, RTD nutritional, soy milk, or shakes and other blended beverages such as milkshakes, smoothies, frappes), nutritional supplement foodstuffs (e.g., high-energy protein bars), confectionery products (e.g., high calcium chews, chewing gum, chocolate, or cookies), dairy products (e.g., yogurt, ice cream, milk, cheese, processed cheese, or butter), and farinaceous products (e.g., bread, muffins, biscuits, cereal or rolls). The relative amount by weight of the nutritional supplement compositions combined with a food product depends on such factors as the density and the serving size of the food product. Typically, the amount of nutritional supplement compositions ranges from 0.1 to about 10 percent by weight, based on the total weight of the food product.

Alternatively, the nutritional supplement composition can be prepared in a form to be directly administered to an individual. By way of example, the composition can be prepared in the form of tablets, chewable tablets, capsules, and liquid syrup.

The formulation of the composition and, if administered via a food product, the amount of the composition blended into the food product, are selected to provide desired amounts of the particular components so as to be effective for controlling weight gain and/or weight loss. By way of example, a typical nutritional supplement composition may be administered to provide between at least about 0.5 and about 6 grams or more of calcium, between at least about 0 and about 10 grams or more of κ-casein fragment 106-169, and between at least about 0 to about 20 grams or more of ACE-inhibiting peptides per serving of the composition. The amount of composition administered can be adjusted as desired to account for differences in physical characteristics and nutritional requirements of the individuals to whom the composition is administered.

In accordance with the methods of the present invention, body weight conditions, including overweight and obesity, are effectively managed and treated. That is, an individual of healthy condition and having a generally ideal weight can manage his weight and maintain a desired weight range. An individual who has a weight in excess of a desired range, and may be considered overweight or obese, can be effectively treated by limiting weight gain and/or promoting weight loss. A therapeutically effective amount of the nutritional supplement composition is administered to an individual to provide these benefits.

EXAMPLES

The following examples further illustrate preferred embodiments of the present invention but are not be construed as in any way limiting the scope of the present invention as set forth in the appended claims.

*Example 1*

This example illustrates preparation of a beverage fortified with a nutritional supplement composition. The components were mixed to yield a product having the composition set forth in Table 2.

*Table 2*

| Ingredient | Amount (weight %) | Weight (grams) |
|---|---|---|
| Water | 70.03 | 350.17 |
| PROVON® 190 | 10.71 | 53.55 |
| Crystalline fructose | 7.00 | 35.00 |
| TRUCAL® FP D7 | 1.48 | 7.38 |
| Carrageenan | 0.08 | 0.40 |
| Maltodextrin | 10.00 | 50.00 |
| Flavor | 0.70 | 3.50 |
| Color | 0.001 | 0.005 |

The liquid ingredients and carrageenan were mixed on high speed for about 5 minutes for hydration. The remaining dry ingredients were blended together and added slowly to the liquid mixture and mixed on low speed for between about 5 to about 10 minutes.

An 11-ounce serving of the fortified drink provides 1 gram of calcium, 30 grams protein, 6 grams of κ-casein fragment 106-169, and 1 gram of ACE-inhibiting peptide components.

*Example 2*

This example illustrates a formulation for a first dry beverage mix fortified with a nutritional supplement composition. The components were mixed to yield a product having the composition set forth in Table 3.

*Table 3*

| Ingredient | Amount (weight %) | Weight (grams) |
|---|---|---|
| PROVON® 190* | 45 | 18.5 |
| TRUCAL®* | 10 | 4 |
| Protein Component/ACE-inhibiting peptide source | 10 | 4 |
| Fructose | 31 | 13 |
| Carrageenan | <1 | 0.3 |
| Dispersion Aids | <1 | 0.3 |
| Flavor | 3 | 1.4 |
| Color | <1 | <0.1 |
| Available from Glanbia Nutritionals Inc., of Monroe, Wisconsin | | |

The dry mix is prepared to be ready for mixing with a sufficient volume of a desired liquid, such as water or skim milk, to provide a beverage.

A single serving (41.5 grams) of the dry mix delivers about 127 calories, about 20 grams of protein, about 1 gram of calcium, about 4 grams of κ-casein fragment 106-169, and about 2 grams of ACE-inhibiting peptide components.

*Example 3*

This example illustrates a formulation for a second dry beverage mix fortified with a nutritional supplement composition. The components were mixed to yield a product having the composition set forth in Table 4.

*Table 4*

| Ingredient | Amount (weight %) | Weight (grams) |
|---|---|---|
| PROVON® 190* | 45 | 18.5 |
| TRUCAL®* | 10 | 4 |
| Protein Component/ACE-inhibiting peptide source | 10 | 4 |
| Fructose | 31 | 13 |
| Carrageenan | <1 | 0.3 |
| Dispersion Aids | <1 | 0.3 |
| Flavor | 4 | 1.8 |
| Color | <1 | <0.1 |
| Available from Glanbia Nutritionals, Inc., of Monroe, Wisconsin. | | |

The dry mix is prepared to be ready for mixing with a sufficient volume of a desired liquid, such as water or skim milk, to provide a beverage.

A single serving (41.5 grams) of the dry mix delivers about 125 calories, about 20 grams of protein, about 1 gram of calcium, about 4 grams of κ-casein fragment 106-169, and about 4 grams of ACE-inhibiting peptide components.

*Example 4*

This example illustrates a formulation for a third dry beverage mix fortified with a nutritional supplement composition. The components were mixed to yield a product having the composition set forth in Table 5.

*Table 5*

| Ingredient | Amount (weight %) | Weight (grams) |
|---|---|---|
| Prolibra* | 54 | 27 |
| Fructose | 27 | 13.3 |
| Maltodextrin | 5 | 2.6 |
| Carrageenan | 1 | 0.5 |
| Flavor | 13 | 6.6 |
| *Available from Glanbia Nutritionals, Inc., of Monroe, Wisconsin | | |

The dry mix is prepared to be ready for mixing with a sufficient volume of a desired liquid, such as water or skim milk, to provide a beverage.

A single serving (50 grams) of the dry mix delivers about 162 calories, about 21 grams of protein, about 1 gram of calcium, about 4 grams of κ-casein fragment 106-169, and about 4 grams of ACE-inhibiting peptide components.

While particular embodiments of the present invention have been described and illustrated, it should be understood that the invention is not limited thereto since modifications may be made by persons skilled in the art. The present application contemplates any and all modifications that fall within the spirit and scope of the underlying invention disclosed and claimed herein.

Preferred embodiments of the invention include the following:

1. A weight control composition comprising:

   a milk mineral blend in an amount effective for decreasing adiposity; and

   a protein compound in an amount effective for enhancing satiety after consumption of food.

2. The composition of embodiment 1, wherein the composition also includes enzyme- inhibiting peptides.

3. The composition of embodiment 2, wherein the enzyme-inhibiting peptides include angiotensin converting enzyme inhibiting peptides.

4. The composition of embodiment 2, wherein at least one of the enzyme-inhibiting peptides limits the breakdown of cholecystokinin.

5. The composition of embodiment 1, wherein the milk mineral blend includes one or more minerals selected from the group consisting of calcium, copper, magnesium, phosphorus, potassium, selenium and zinc for providing a healthy diet.

6. The composition of embodiment 3, wherein the milk mineral blend includes one or more minerals selected from the group consisting of calcium, copper, magnesium, phosphorus, potassium, selenium and zinc for providing a healthy diet.

7. The composition of embodiment 1, wherein the protein source is selected from the group consisting of milk-derived protein products, whey-derived protein products, soy-derived protein products and combinations thereof.

8. The composition of embodiment 1, wherein the composition is administered between meals.

9. The composition of embodiment 1, wherein the composition is administered as part of a meal.

10. A nutritional supplement for treatment of obesity comprising:

   calcium in an amount effective for decreasing adiposity; and

   κ-casein fragment 106-169 in an amount effective for stimulating satiety.

11. The supplement of embodiment 10, wherein the supplement includes at least about 0.5 grams per serving of the supplement of calcium.

12. The supplement of embodiment 10, wherein the supplement includes at least about 3 grams per serving of the supplement of κ-casein fragment 106-169.

13. The supplement of embodiment 10, wherein the supplement includes enzyme-inhibiting peptides effective for enhancing fat metabolism.

14. The supplement of embodiment 13, wherein the peptides include angiotensin converting enzyme inhibiting peptides.

15. The supplement of embodiment 10, wherein the supplement includes enzyme-inhibiting peptides effective for limiting the breakdown of cholecystokinin.

16. The supplement of embodiment 10, wherein the calcium is derived from milk mineral.

17. The supplement of embodiment 10, wherein the supplement is in the form of a dry powder.

18. The supplement of embodiment 10, wherein the supplement is administered through a food product that has been fortified with the supplement.

19. A method of treating obesity comprising administering to an individual a nutritional composition having a therapeutically effective amount of milk mineral and protein component.

20. The method of embodiment 19, wherein the milk mineral is effective for decreasing adiposity and the protein component is effective for enhancing satiety after consumption of food.

21. The method of embodiment 20, wherein the composition includes enzyme-inhibiting peptides effective for enhancing fat metabolism.

22. The method of embodiment 21, wherein the peptides are angiotensin converting enzyme inhibiting peptides.

23. The method of embodiment 19, wherein the composition is added to a food product that is consumed by the individual.

24. The method of embodiment 23, wherein the milk mineral includes calcium and at least about 0.5 grams of calcium is provided per serving of the food product.

25. The method of embodiment 23, wherein the protein component includes κ-casein fragment 106-169 and at least about 3 grams of κ-casein fragment 106-169 is provided per serving of the food product.

26. The method of embodiment 23, wherein the food product is selected from the group consisting of acidic juice beverages, acidic beverages, neutral pH beverages, high-energy protein bars, confectionery products, dairy products, and farinaceous products.

27. A weight management composition comprising therapeutically effective amounts of two or more weight control components selected from the group consisting of milk mineral blend, protein compound and enzyme-inhibiting peptides.

28. The composition of embodiment 27, wherein the milk mineral blend includes one or more minerals selected from the group consisting of calcium, copper, magnesium, phosphorus, potassium, selenium and zinc for providing a healthy diet.

29. The composition of embodiment 27, wherein the protein source is selected from the group consisting of milk-derived protein products, whey-derived protein products, soy-derived protein products and combinations thereof.

30. The composition of embodiment 27, wherein the enzyme-inhibiting peptides include angiotensin converting enzyme inhibiting peptides.

31. The composition of embodiment 27, wherein the composition is administered to an individual between meals.

32. The composition of embodiment 27, wherein the composition is administered to an individual as part of a meal.

33. The composition of embodiment 27, wherein the composition is blended with a food product.

34. A method of treating body weight conditions comprising administering to an individual a therapeutically effective amount of a nutritional composition having two or more weight control components selected from the group consisting of milk mineral blend, protein compound, and enzyme-inhibiting peptides.

35. The method of embodiment 34, wherein the milk mineral blend is administered in an amount effective for decreasing adiposity.

36. The method of embodiment 34, wherein the protein compound is administered in an amount effective for enhancing satiety after consumption of food.

37. The method of embodiment 34, wherein the enzyme-inhibiting peptides are administered in an amount effective for enhancing fat metabolism.

38. The method of embodiment 37, wherein the peptides are angiotensin converting enzyme inhibiting peptides.

39. The method of embodiment 34, wherein the composition is added to a food product that is consumed by the individual.

40. The method of embodiment 39, wherein the food product is selected from the group consisting of acidic juice beverages, acidic beverages, neutral pH beverages, nutritional supplement foodstuffs, confectionery products, dairy products, and farinaceous products.

41. A nutritional supplement for management, treatment or prevention of body weight conditions comprising therapeutically effective amounts of two or more components selected from the group consisting of milk mineral blend, protein compound and enzyme-inhibiting peptides.

42. The supplement of embodiment 41, wherein the supplement includes milk mineral blend having calcium in an amount effective for decreasing adiposity.

43. The supplement of embodiment 42, wherein the calcium is derived from milk mineral.

44. The supplement of embodiment 41, wherein the protein compound includes κ-casein fragment 106-169 in an amount effective for stimulating satiety.

45. The supplement of embodiment 41, wherein the supplement includes enzyme-inhibiting peptides in an amount effective for enhancing fat metabolism.

46. The supplement of embodiment 45, wherein the peptides include angiotensin converting enzyme inhibiting peptides.

47. The supplement of embodiment 41, wherein the supplement includes enzyme-inhibiting peptides effective for limiting the breakdown of cholecystokinin.

48. The supplement of embodiment 41, wherein the supplement is in the form of a dry powder.

49. The supplement of embodiment 41, wherein the supplement is administered through a food product that has been fortified with the supplement.

**Claims**

1. A weight management composition comprising therapeutically effective amounts of two or more weight control components selected from the group consisting of:

   milk mineral blend (preferably wherein the milk mineral blend includes one or more minerals selected from the group consisting of calcium, copper, magnesium, phosphorus, potassium, selenium and zinc for providing a healthy diet);
   protein compound (preferably wherein the protein source is selected from the group consisting of milk-derived protein products, whey-derived protein products, soy-derived protein products and combinations thereof); and
   enzyme-inhibiting peptides (preferably wherein the enzyme-inhibiting peptides include angiotensin converting enzyme inhibiting peptides).

2. The composition of Claim 1, wherein the composition is administered to an individual between meals or as part of a meal.

3. The composition of Claim 1, wherein the composition is blended with a food product.

4. A nutritional composition for use in treating body weight conditions having two or more weight control components selected from the group consisting of:

   milk mineral blend;
   protein compound;
   and enzyme-inhibiting peptides.

5. The composition of Claim 4 for use in treating body weight conditions, wherein the milk mineral blend is administered in an amount effective for decreasing adiposity.

6. The composition of Claim 4 for use in treating body weight conditions, wherein the protein compound is administered in an amount effective for enhancing satiety after consumption of food.

7. The composition of Claim 4 for use in treating body weight conditions, wherein the enzyme-inhibiting peptides are administered in an amount effective for enhancing fat metabolism, preferably wherein the peptides are angiotensin converting enzyme inhibiting peptides.

8. The composition of Claim 4 for use in treating body weight conditions, wherein the composition is added to a food product that is consumed by the individual, preferably wherein the food product is selected from the group consisting of acidic juice beverages, acidic beverages, neutral pH beverages, nutritional supplement foodstuffs, confectionery products, dairy products, and farinaceous products.

9. A nutritional supplement for management, treatment or prevention of body weight conditions comprising therapeutically effective amounts of two or more components selected from the group consisting of:

   milk mineral blend;
   protein compound;
   and enzyme- inhibiting peptides.

10. The supplement of Claim 9, wherein the supplement includes milk mineral blend having calcium in an amount effective for decreasing adiposity, preferably wherein the calcium is derived from milk mineral.

11. The supplement of Claim 9, wherein the protein compound includes κ-casein fragment 106-169 in an amount effective for stimulating satiety.

12. The supplement of Claim 9, wherein the supplement includes enzyme-inhibiting peptides in an amount effective for enhancing fat metabolism, preferably wherein the peptides include angiotensin converting enzyme inhibiting peptides.

13. The supplement of Claim 9, wherein the supplement includes enzyme-inhibiting peptides effective for limiting the breakdown of cholecystokinin.

**14.** The supplement of Claim 9, wherein the supplement is in the form of a dry powder.

**15.** The supplement of Claim 9, wherein the supplement is administered through a food product that has been fortified with the supplement.

**Europäisches Patentamt**
**European Patent Office**
**Office européen des brevets**

# EUROPEAN SEARCH REPORT

Application Number

EP 10 01 1956

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 207 638 B1 (PORTMAN ROBERT) 27 March 2001 (2001-03-27) * column 1, line 42 - column 2, line 47 * * column 5, line 15 - column 8, line 35 * ----- | 1-15 | INV. A61P3/04 A23L1/29 A23L1/304 |
| X | US 6 340 669 B1 (CESTARO BENVENUTO ET AL) 22 January 2002 (2002-01-22) * column 1, line 16 - line 25; claim 21; example 5 * ----- | 1-15 | A23L1/305 A61K35/20 A61K38/00 A61K38/55 |
| X | US 4 833 128 A (SOLOMON NEIL ET AL) 23 May 1989 (1989-05-23) * column 2, line 22 - column 4, line 22 * ----- | 1-15 | |
| X | WO 99/26971 A (BRIEN SIMPSON NEIL MARTIN O ;DASHPER STUART GEOFFREY (AU); REYNOLD) 3 June 1999 (1999-06-03) * examples 4,8 * ----- | 1-15 | |
| X | US 6 106 874 A (LIEBRECHT JEFFERY WAYNE ET AL) 22 August 2000 (2000-08-22) * column 7, line 39 - line 58; claims * ----- | 1-15 | TECHNICAL FIELDS SEARCHED (IPC) |
| X | ANONYMOUS: "DRINK THIS! NEW STUDY FINDS LOWFAT MILK MAY HELP REDUCE OBESITY RISK", NATIONAL DAIRY COUNCIL, [Online] XP002242622, Retrieved from the Internet: URL:http://www.nationaldairycouncil.org/lv 104/newsres/releases/press_releases6GKQIK. asp> [retrieved on 2003-05-27] * the whole document * ----- | 1-15 | A23L A61K A61P |

-/--

The present search report has been drawn up for all claims

| Place of search | Date of completion of the search | Examiner |
|---|---|---|
| Munich | 31 August 2011 | Rinaldi, Francesco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**

**European Patent Office**

**Office européen des brevets**

# EUROPEAN SEARCH REPORT

Application Number

EP 10 01 1956

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZEMEL M B ET AL: "REGULATION OF ADIPOSITY BY DIETARY CALCIUM", FASEB JOURNAL, FED. OF AMERICAN SOC. FOR EXPERIMENTAL BIOLOGY, BETHESDA, MD, US, vol. 14, no. 9, June 2000 (2000-06), pages 1132-1138, XP001096070, ISSN: 0892-6638 * abstract * * page 1137, left-hand column, last paragraph - right-hand column, paragraph 1 * | 1-15 | |
| X,P | WO 02/071854 A (UNILEVER PLC ;LEVER HINDUSTAN LTD (IN); UNILEVER NV (NL)) 19 September 2002 (2002-09-19) * claims * | 1-15 | |
| T | DATABASE MEDLINE [Online] April 2002 (2002-04), ZEMEL MICHAEL B: "Regulation of adiposity and obesity risk by dietary calcium: mechanisms and implications.", XP002242591, Database accession no. NLM11999543 * abstract * & JOURNAL OF THE AMERICAN COLLEGE OF NUTRITION. UNITED STATES APR 2002, vol. 21, no. 2, April 2002 (2002-04), pages 146S-151S, ISSN: 0731-5724 | 1-15 | TECHNICAL FIELDS SEARCHED (IPC) |

-/--

The present search report has been drawn up for all claims

| Place of search | Date of completion of the search | Examiner |
|---|---|---|
| Munich | 31 August 2011 | Rinaldi, Francesco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 01 1956

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MASUO K ET AL: "EFFECTS OF WEIGHT REDUCTION, ACE INHIBITOR AND ANGIOTENSIN II ANTAGONIST ON OBESITY HYPERTENSION", AMERICAN JOURNAL OF HYPERTENSION, NEW YORK, NY, US, vol. 13, no. 4, PART 2, April 2000 (2000-04), page 54A, XP001071074, * abstract * | 1-15 | |

-----

TECHNICAL FIELDS
SEARCHED    (IPC)

The present search report has been drawn up for all claims

| Place of search | Date of completion of the search | Examiner |
|---|---|---|
| Munich | 31 August 2011 | Rinaldi, Francesco |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                 EP 10 01 1956

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2011

| Patent document cited in search report | | Publication date | Patent family member(s) | | Publication date |
|---|---|---|---|---|---|
| US 6207638 | B1 | 27-03-2001 | AU | 4176201 A | 03-09-2001 |
| | | | CA | 2400312 A1 | 30-08-2001 |
| | | | EP | 1259112 A1 | 27-11-2002 |
| | | | JP | 2003523368 A | 05-08-2003 |
| | | | WO | 0162086 A1 | 30-08-2001 |
| | | | US | 6468962 B1 | 22-10-2002 |
| | | | US | 2001021694 A1 | 13-09-2001 |
| | | | US | 2002019334 A1 | 14-02-2002 |
| US 6340669 | B1 | 22-01-2002 | AT | 271392 T | 15-08-2004 |
| | | | AU | 2542500 A | 07-08-2000 |
| | | | CA | 2360065 A1 | 27-07-2000 |
| | | | DE | 60012301 D1 | 26-08-2004 |
| | | | WO | 0043036 A2 | 27-07-2000 |
| | | | EP | 1143999 A2 | 17-10-2001 |
| | | | JP | 2002535288 A | 22-10-2002 |
| US 4833128 | A | 23-05-1989 | NONE | | |
| WO 9926971 | A | 03-06-1999 | AT | 368053 T | 15-08-2007 |
| | | | CA | 2311389 A1 | 03-06-1999 |
| | | | DE | 69838143 T2 | 10-04-2008 |
| | | | EP | 1032592 A1 | 06-09-2000 |
| | | | HK | 1030551 A1 | 28-12-2007 |
| | | | JP | 2002503641 A | 05-02-2002 |
| | | | ZA | 9810679 A | 30-07-1999 |
| US 6106874 | A | 22-08-2000 | CA | 2350501 A1 | 25-05-2000 |
| | | | EP | 1130979 A1 | 12-09-2001 |
| | | | JP | 2002529110 A | 10-09-2002 |
| | | | WO | 0028838 A1 | 25-05-2000 |
| WO 02071854 | A | 19-09-2002 | AU | 2002302385 B2 | 06-10-2005 |
| | | | EP | 1365656 A1 | 03-12-2003 |
| | | | US | 2002182301 A1 | 05-12-2002 |
| | | | ZA | 200306437 A | 19-08-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 60360709 B
- US 6384087 B, Zemel
- US 6207638 B, Portman
- US 5639501 A
- US 5278288 A
- US 5280107 A

### Non-patent literature cited in the description

- **SHI et al.** Effects of dietary calcium on adipocyte lipid metabolism and body weight regulation in energy-restricted aP2-agouti transgenic mice. *FASEB J.,* 2001, vol. 15 (2), 291-93
- **ZEMEL et al.** Regulation of adiposity by dietary calcium. *FASB J.,* 2000, vol. 14 (9), 1132-38
- **RANHOTRA et al.** Bioavailability of Calcium in a High Calcium Whey Fraction. *Nutrition Research,* 1997, vol. 17 (11-12), 1663-1670